# FASCICULE DE BREVET EUROPEEN

(11) **EP 3 558 111 B1**
(45) Date de publication et mention de la délivrance du brevet: **20.08.2025**
(21) Numéro de dépôt: 17829265.2
(22) Date de dépôt: 20.12.2017
(51) Int. Cl.: A61B 5/055, G05D 21/00, G01N 23/18, G05D 21/02, G16Z 99/00

(54) **PROCEDE POUR MAINTENIR UN EQUILIBRE D'UN PARAMETRE PHYSICO-CHIMIQUE D'UN MILIEU, PRODUIT PROGRAMME D'ORDINATEUR ET MODULE ELECTRONIQUE ASSOCIES**
VERFAHREN ZUR AUFRECHTERHALTUNG EINES GLEICHGEWICHTS EINES PHYSIKALISCH-CHEMISCHEN PARAMETERS EINES MEDIUMS, ZUGEHÖRIGES COMPUTERPROGRAMMPRODUKT UND ELEKTRONISCHES MODUL
METHOD FOR MAINTAINING AN EQUILIBRIUM OF A PHYSICO-CHEMICAL PARAMETER OF A MEDIUM, ASSOCIATED COMPUTER PROGRAM PRODUCT AND ELECTRONIC MODULE

(30) Priorité: 20.12.2016 FR 1662813
(43) Date de publication de la demande: 30.10.2019
(73) Titulaire: Ondilo, 13760 Saint-Cannat (FR)
(72) Inventeur: FIORINI, Nicolas, 13330 Pelissanne (FR); ROUSSEL, Didier, 13330 Pelissanne (FR)
(74) Mandataire: Brun, Philippe Alexandre Georges
(86) Numéro de dépôt international: PCT/FR2017/053747
(87) Numéro de publication internationale: WO 2018/115751

(56) Documents cités:
- EP-A1- 2 273 039
- WO-A1-2010/052419
- WO-A1-2015/033032
- WO-A1-2015/082863
- WO-A1-2016/151232
- WO-A2-2004/029625
- FR-A1- 2 969 281
- FR-A1- 2 981 474
- HECTOR RICARDO HERNANDEZ DE LEON ET AL: "Supervision et diagnostic des procédés de production d'eau potable", 27 September 2006 (2006-09-27), XP055412174, Retrieved from the Internet <URL:https://tel.archives-ouvertes.fr/tel-00136157/document> [retrieved on 20171005]

## Description

L'invention concerne un procédé mis en œuvre par ordinateur pour maintenir un équilibre d'un ou plusieurs paramètres physico-chimiques caractérisant un milieu et pour recouvrer ledit équilibre en cas de perte de ce dernier. Plus précisément, l'invention concerne un procédé permettant d'estimer la pertinence d'une action corrective sur ledit milieu dans le but de recouvrer un équilibre. Une telle action corrective peut, à titre d'exemple avantageux mais non limitatif, consister en un apport d'un agent chimique, dont le dosage est ajusté au milieu considéré, prévenant ainsi toute surconsommation inutile ou néfaste pour ledit milieu.

L'invention sera décrite, de manière préférée mais non limitative, au travers d'un exemple d'application relative au maintien de l'équilibre de l'eau de baignade d'une piscine. L'invention ne saurait toutefois être limitée à un tel milieu, ce dernier pouvant consister en un espace de culture d'un jardin, du terreau, etc.

Pour assurer une baignade saine et un fonctionnement pérenne des équipements d'une piscine, l'eau de baignade de celle-ci doit satisfaire plusieurs critères liés à la désinfection de l'eau et à l'équilibre de cette dernière. En effet, l'eau d'une piscine est par définition impure, c'est-à-dire qu'elle est composée, outre les molécules d'eau, de plusieurs éléments chimiques, tels qu'à titre d'exemples non limitatifs du calcium, du magnésium, du sodium, etc. La quantité non maitrisée de tels éléments chimiques au sein de l'eau d'une piscine peut ainsi, dans certains cas, perturber l'équilibre de ladite eau de piscine. A titre d'exemple non limitatif, une augmentation de la proportion de calcium peut déséquilibrer une telle composition et entraîner un dépôt de calcaire sur les équipements de la piscine, tels qu'à titre d'exemple sur les canalisations et/ou le revêtement du bassin, etc. Un tel dépôt de calcaire peut alors endommager lesdits équipements et troubler l'eau. Par ailleurs, l'eau d'une piscine est aussi généralement le territoire de microorganismes plus ou moins nocifs pour un baigneur. A titre d'exemples non limitatifs, de tels microorganismes peuvent consister en des algues, des bactéries, des champignons, etc. Lesdits microorganismes se nourrissent principalement de déchets organiques apportés par l'environnement extérieur, tels que, par exemple, la poussière, le pollen, les insectes, etc., ou introduits par des baigneurs, tels que par exemple, des déchets ou éléments véhiculés par la sueur, la peau, les produits de beauté, etc. De par la présence de nombreux microorganismes au sein de l'eau de la piscine, lesdits microorganismes prolifèrent, entraînant aussi un déséquilibre microbiologique. Un tel déséquilibre peut, par exemple, se traduire par une prolifération incontrôlée d'algues dans la piscine. Un dépôt d'algues peut alors apparaître sur les parois de ladite piscine et l'eau peut prendre une teinte verte, peu engageante pour les baigneurs quand bien même aguerris.

Pour assainir l'eau et recouvrer un équilibre, plusieurs actions correctives peuvent ainsi être mises en œuvre. A titre d'exemple non limitatif, une première action peut consister à filtrer l'eau pour retirer du milieu des déchets solides apportés par l'environnement extérieur et les baigneurs, au moyen, par exemple, de l'utilisation manuelle d'un filet ou d'un actionnement automatique d'un automate de filtration (également connu sous la terminologie anglo-saxonne « *skimmer* »)*.* Bien que ce type d'actions permette de retirer tout ou partie des déchets solides présents au sein de l'eau d'une piscine, il ne permet pas de supprimer les microorganismes, ces derniers étant généralement trop petits pour être retenus dans de tels filtres, et ainsi désinfecter proprement l'eau. Pour désinfecter proprement l'eau, c'est-à-dire supprimer les microorganismes, il est nécessaire d'utiliser, en variante ou en complément, des produits ou agents chimiques, tels que, par exemple, le chlore et/ou des algicides. De tels produits chimiques peuvent être introduits manuellement ou automatiquement.

L'équilibre d'une piscine peut être estimé et régulé à partir de plusieurs grandeurs physiques, tel qu'à titre d'exemple non limitatif, le potentiel Hydrogène communément appelé « pH ». Ce dernier permet d'établir l'acidité ou la basicité d'un milieu, c'est-à-dire si le milieu est basique, neutre ou acide. A titre d'exemple non limitatif, l'eau d'une piscine a idéalement un pH compris entre 6,9 et 7,6. La mesure d'un tel pH permet de :
- renforcer l'efficacité des agents désinfectants, tel que par exemple le chlore ;
- diminuer l'irritation de la peau et des yeux des baigneurs ;
- éviter la corrosion des équipements métalliques employés en lien avec le milieu;
- limiter la précipitation de sels de calcium au sein de l'eau d'une piscine, de tels sels de calcium pouvant provoquer l'entartrage des équipements de la piscine, etc.

La mesure du pH peut varier en fonction du type de désinfectant employé, de la température de l'eau, de la pluie, de la fréquentation du bassin, etc. Le pH idéal de l'eau d'une piscine peut en outre dépendre du type de revêtement de ladite piscine. Pour réguler la valeur de la mesure du pH, il est possible d'introduire des produits ou agents chimiques, permettant de diminuer ou d'augmenter la valeur de la mesure dudit pH, afin finalement d'atteindre une valeur comprise entre 6,9 et 7, 6.

L'équilibre d'une piscine peut en outre être estimé à partir de la valeur d'un paramètre appelé « TAC », acronyme de Titre Alcalimétrique Complet, reflétant l'alcalinité de l'eau, c'est-à-dire la concentration dans l'eau des ions carbonates et hydrogénocarbonates, ou encore à partir de la valeur d'un paramètre appelé « TH », acronyme de Titre Hydrotimétrique, reflétant la dureté de l'eau, c'est-à-dire la concentration en calcium et en magnésium de l'eau, indiquant la minéralisation de l'eau. Idéalement, la valeur du TAC est comprise entre quatre-vingt (80) et cent vingt (120) milligrammes par litre et celle du TH, entre cent (100) et deux cent cinquante (250) milligrammes par litre. Les valeurs du TAC et/ou du pH influent directement sur la valeur du pH ou encore sur les propriétés physico-chimiques de l'eau d'un bassin. Ainsi, lorsque la valeur du TAC est trop basse, le pH devient instable, c'est-à-dire qu'il peut varier rapidement d'un état basique à un état acide sans pouvoir se stabiliser correctement dans l'un de ces états. Quant au TH, lorsqu'il est trop bas, il peut provoquer une irritation des yeux et de la peau des baigneurs. Au contraire, lorsque les valeurs respectives TAC et/ou de TH sont trop élevées, l'eau peut devenir trouble et un dépôt de tarte et de calcaire peut être observé sur les parois et dans les conduits des équipements de filtration ou de chauffage de l'eau de la piscine, par exemple.

Le pouvoir désinfectant d'une piscine, c'est-à-dire la quantité d'agent(s) chimique(s) désinfectant(s) actif(s) dans l'eau de ladite piscine, peut être déterminé à partir de la valeur du potentiel d'oxydoréduction, également qualifié de potentiel redox, de ladite eau, généralement exprimé en millivolts,. Un tel potentiel permet de déterminer le potentiel oxydant d'un agent chimique désinfectant, permettant ainsi de prévoir la réactivité dudit agent chimique désinfectant. A titre d'exemples non limitatifs, un potentiel redox élevé peut signifier une concentration d'agent chimique désinfectant importante dans l'eau de baignade, alors qu'un potentiel redox faible peut signifier une concentration d'agent chimique désinfectant faible. Egalement, à titre d'exemples non limitatifs, une eau de piscine peut être considérée comme désinfectée, lorsque le potentiel redox est supérieur ou égal à six cent cinquante (650) millivolts. Par ailleurs, l'oxydation des agents chimiques désinfectants, et donc leur pouvoir désinfectant, peut dépendre de la valeur du pH et de la valeur du TAC courantes dans le milieu.

L'équilibre d'une piscine peut donc être estimé à partir de plusieurs paramètres, grandeurs et/ou mesures physico-chimiques, de tels paramètres, grandeurs et/ou mesures physico-chimiques influant, comme précédemment décrit, les uns ou les unes sur les autres.

Pour maintenir l'équilibre de l'eau de sa piscine, un propriétaire, un baigneur ou plus généralement un opérateur peut contrôler périodiquement différents paramètres physico-chimiques de ladite piscine, par exemple une fois par jour au moyen de testeurs de paramètre(s) ou plus généralement d'une trousse ou d'un kit d'analyse permettant d'estimer notamment la teneur en chlore de la piscine, la valeur du pH et la valeur du TAC. De tels kits d'analyse peuvent se présenter sous la forme de bandelettes dédiées à un paramètre physico-chimique déterminé, lesdites bandelettes devant être insérées dans l'eau de la piscine. Une telle bandelette peut ainsi, par exemple, être dédiée à l'estimation du pH. Au contact de l'eau, ladite bandelette se colore par réaction chimique entre ladite bandelette et l'eau de la piscine. La bandelette ainsi colorée peut alors être comparée à une table de couleurs prédéterminée en fonction de taux de pH type prédéfinis. Le propriétaire ou l'utilisateur d'une piscine peut ainsi estimer la valeur de pH de sa piscine. Bien que permettant d'obtenir rapidement et facilement la valeur courante du pH de l'eau d'une piscine, l'emploi de telles bandelettes présente néanmoins plusieurs inconvénients, comme par exemple une imprécision, la table de couleurs étant généralement limitée, et une durée de vie limitée dans le temps puisque les bandelettes périment au bout d'un certain temps.

En variante, une autre méthode peut consister à utiliser un testeur électronique ou une sonde, par exemple, de pH ou de potentiel d'oxydo-réduction. La valeur courante de la mesure délivrée par un tel testeur électronique ou une telle sonde peut ainsi être affichée par ledit testeur électronique. Toutefois, bien que très pratiques, de tels testeurs électroniques nécessitent d'être étalonnés à partir de solutions tampons. De nombreuses erreurs peuvent ainsi survenir en cas de mauvaise étalonnage desdits testeurs ou sondes, entraînant une interprétation de la valeur du pH erronée, lors de prises de mesures, et pouvant alors engendrer une ou plusieurs actions correctives inappropriées.

Une fois la valeur courante d'un paramètre physico-chimique d'une piscine connue, telle que par exemple, celle du pH, un utilisateur, un propriétaire ou un opérateur de la piscine peut ajouter manuellement un agent chimique déterminé permettant de rétablir son équilibre. Par exemple, si la valeur du pH mesurée est supérieure à 7,6, l'eau de la piscine est considérée comme trop basique. Il est donc nécessaire d'y ajouter un agent chimique permettant de faire baisser ledit pH et rendre l'eau de la piscine neutre, c'est-à-dire à une valeur de pH proche de ou sensiblement égale à 7, ou à une valeur de pH compris entre 6,9 et 7,6. Au contraire, si la valeur du pH est inférieure à 6,9, l'eau de la piscine est acide. Il faut alors y ajouter un agent chimique permettant d'augmenter ledit pH et de rendre l'eau de la piscine neutre.

En variante, selon une deuxième méthode, la gestion de la concentration d'un ou plusieurs agents chimiques désinfectants ou plus généralement des mesures des valeurs respectives d'un ou plusieurs paramètres physico-chimique d'une piscine peut être effectuée par un ou plusieurs automates mesurant des valeurs courantes desdits paramètres, et introduisant de manière automatique le ou lesdits agents chimiques selon une distance mesurée entre la valeur nominale d'un paramètre physico-chimique donné au regard de la valeur mesurée.

Toutefois, les bandelettes ou les automates précédemment décrits présentent plusieurs inconvénients. bandelettes ou de tels automates ne prend pas en compte certaines interactions entre paramètres. Par exemple, un pH trop acide peut réduire l'efficacité d'un agent chimique désinfectant, entrainant un risque de surdosage de l'agent chimique désinfectant alors qu'il aurait pu suffire d'accroitre, en lieu et place, la concentration d'un agent chimique amplificateur de pH. En outre, lors d'une introduction manuelle d'un ou plusieurs agents chimiques dans le milieu considéré, un opérateur doit généralement calculer par lui-même la quantité d'agent chimique à introduire. Dans le cas d'une piscine, cette quantité peut dépendre de la taille du bassin, de la concentration de l'agent chimique, etc. Pour une piscine d'un particulier, un propriétaire peut être souvent désemparé ou peu en capacité d'effectuer le calcul approprié. Ainsi, lors dudit calcul de quantité, de nouvelles erreurs peuvent survenir, pouvant après l'introduction dudit agent, rompre l'équilibre de la piscine et, par exemple, altérer des équipements de la piscine, par un taux de chlore trop élevé par exemple.

Les demandes de brevet WO2010/052419 et EP 2 273 039 divulguent des dispositifs automatiques de régulation de paramètres phisico-chimique d'un milieu tel qu'une piscine. Ces documents effectuent une mesure de paramètres de la piscine afin de déterminer si un ajout d'un ou plusieurs agents de correction est à réaliser dans ladite piscine.

Les méthodes usuelles présentées ci-dessus permettent de réguler certains paramètres physico-chimiques de l'eau d'une piscine à un instant donné, sans prendre en compte l'ensemble des paramètres de la piscine. Une piscine peut dès lors être saturée en agent chimique sans qu'on ne le sache. L'ajout d'un agent chimique peut ainsi se révéler sans effet, le propriétaire tentant de corriger un paramètre par une introduction disproportionnée et inefficace d'un agent chimique.

L'invention permet de répondre à tout ou partie des inconvénients soulevés par les solutions connues en proposant un procédé innovant de maintien de l'équilibre d'un milieu, tel qu'une piscine.

Un tel procédé procure de nombreux avantages, parmi lesquels nous pouvons mentionner qu'il permet :
- une prise en compte d'une pluralité de paramètres physico-chimiques d'un milieu ;
- une adaptation automatique et dynamique d'actions correctives aux comportements d'un milieu en particulier ;
- une anticipation de l'impact de certains facteurs, tels qu'une sur-fréquentation ou des intempéries, sur l'équilibre du milieu, minimisant ainsi certaines actions correctives si celles-ci n'étaient que purement réactives ;
- une adaptation desdites actions quant à l'environnement du milieu, au regard par exemple de l'exposition ou de la végétation des environs ;
- un accompagnement de l'utilisateur lors d'un ajout manuel d'un ou plusieurs agents chimiques ;
- une gestion des ressources optimisées par l'utilisation du système selon un environnement client serveur ;
- une utilisation pérenne des équipements du milieu.

A cette fin, il est notamment prévu un procédé pour maintenir un équilibre d'un paramètre physico-chimique d'un milieu, ledit procédé étant mis en œuvre par une unité de traitement d'un système de régulation dudit milieu, ladite unité de traitement coopérant avec des moyens de mémorisation enregistrant des données en lien avec un paramètre de milieu. Les moyens de mémorisation comportent dès lors un premier enregistrement dédié audit paramètre de milieu et agencé pour mémoriser une valeur courante dudit paramètre et une valeur nominale. Pour réguler un tel système, ledit procédé comporte :
- une étape pour lire la valeur courante dudit paramètre ;
- une étape pour lire la valeur nominale dudit paramètre ;
- une étape pour estimer une distance entre ladite valeur nominale et la valeur courante ;
- une étape pour déclencher une action selon la valeur de ladite distance.

Pour maintenir l'équilibre d'un paramètre physico-chimique dudit milieu, l'enregistrement dédié audit paramètre de milieu comporte un champ pour mémoriser une date d'échéance pour que la valeur courante recouvre la valeur nominale, et ledit procédé comporte :
- une étape, préalable à l'étape pour déclencher une action, pour lire la valeur du champ pour mémoriser la date d'échéance ;
- l'étape pour déclencher une action selon ladite distance étant mise en œuvre si et seulement si la valeur du champ pour mémoriser la date d'échéance consiste en une valeur prédéterminée caractérisant l'absence de définition de la date d'échéance pour que la valeur courante recouvre la valeur nominale.

Préférentiellement mais non limitativement, un procédé mis en œuvre par ordinateur maintenir un équilibre d'un paramètre physico-chimique d'un milieu conforme à l'invention peut comporter une étape antérieure à l'étape pour déclencher une action, pour élaborer ladite action selon la valeur de ladite distance.

Selon un mode de réalisation avantageux mais non limitatif, l'étape pour élaborer une action dudit procédé peut comporter une étape pour estimer une valeur nominale dudit paramètre.

En variante ou en complément, l'étape pour élaborer une action dudit procédé peut comporter une étape pour définir une date d'échéance pour que la valeur courante recouvre la valeur nominale selon ladite action.

En outre, l'étape pour déclencher une action d'un procédé pour maintenir un équilibre d'un paramètre physico-chimique d'un milieu conforme à l'invention peut consister à mettre à jour l'enregistrement dédié audit paramètre pour y inscrire la valeur nominale dudit paramètre ou la date d'échéance pour que la valeur courante recouvre la valeur nominale selon ladite action.

Pour mettre en oeuvre une action déterminée par l'unité de traitement du système de régulation, cette dernière peut coopérer avec un actionneur. L'étape pour déclencher une action d'un procédé conforme à l'invention peut dès lors consister à élaborer une commande à destination dudit actionneur pour mettre en œuvre l'action selon la distance estimée.

Pour avertir un utilisateur d'un système de régulation conforme à l'invention d'une action à effectuer, l'unité de traitement peut en outre coopérer avec une interface homme-machine de restitution. L'étape pour déclencher une action d'un procédé conforme à l'invention peut dès lors consister à élaborer une consigne à destination dudit utilisateur et déclencher la restitution de ladite consigne par l'interface homme-machine de restitution.

Pour répercuter les effets d'une action associée sur tout ou partie des paramètres du système, les moyens de mémorisation peuvent comporter un deuxième enregistrement dédié à un deuxième paramètre de milieu et agencé pour mémoriser une valeur nominale et une valeur courante dudit deuxième paramètre. Le procédé pour maintenir un équilibre d'un paramètre physico-chimique d'un milieu peut dès lors comporter une étape, postérieure à l'étape pour élaborer une action, pour estimer une valeur nominale et/ou une valeur courante dudit deuxième paramètre selon l'action précédemment élaborée.

Pour acquitter une échéance d'action, un procédé conforme à l'invention peut également comporter une étape consistant à inscrire, dans le champ pour mémoriser une date d'échéance, une valeur prédéterminée caractérisant l'absence de définition de la date d'échéance, ladite étape étant mise en œuvre si la valeur de la distance entre ladite valeur nominale et la valeur courante atteste d'une distance sensiblement nulle.

Lorsqu'une action n'a pas eu l'effet escompté, un procédé pour maintenir un équilibre d'un paramètre physico-chimique d'un milieu conforme à l'invention peut comporter une étape de correction d'une action, antérieure à l'étape pour déclencher une action, ladite étape de correction étant mise en œuvre si et seulement si :
∘ la date d'échéance pour que la valeur courante recouvre la valeur nominale soit atteinte ;
∘ la valeur de la distance entre ladite valeur nominale et la valeur courante est non-nulle.

Selon un exemple de réalisation avantageux mais non limitatif, l'étape de correction d'une action dudit procédé peut comporter une étape pour estimer une nouvelle valeur nominale dudit paramètre.

Préférentiellement mais non limitativement, l'étape de correction d'une action dudit procédé peut, en outre, comporter une étape pour définir la date d'échéance pour que la valeur courante recouvre la valeur nominale selon ladite correction d'une action.

Par ailleurs, en variante ou en complément, les moyens de mémorisation d'un système de régulation peuvent en outre comporter une valeur courante d'une période précédente. Pour limiter ou éviter une trop grande réactivité ou variabilité d'un paramètre, l'étape pour lire la valeur courante d'un procédé pour maintenir un équilibre d'un paramètre physico-chimique d'un milieu conforme à l'invention peut, dès lors, consister à lire la valeur précédente et calculer une nouvelle valeur courante à partir desdites valeurs lues.

Pour anticiper un événement perturbateur pouvant altérer un équilibre, les moyens de mémorisation peuvent également comporter une donnée d'anticipation. L'étape pour lire la valeur courante dudit procédé peut alors consister en outre à lire la donnée d'anticipation et calculer une nouvelle valeur courante du paramètre à partir de ladite valeur d'anticipation.

Pour connaître la valeur courante d'un paramètre, l'unité de traitement peut également coopérer avec des moyens de capture. Ledit procédé peut comporter ainsi une étape, préalable à l'étape pour lire la valeur courante dudit paramètre, pour déclencher l'acquisition de ladite valeur par lesdits moyens de capture et inscrire ladite valeur comme valeur courante.

Selon un deuxième objet, l'invention concerne un produit programme d'ordinateur comportant une ou plusieurs instructions de programme exploitables par une unité de moyens de capture. Ledit procédé peut comporter ainsi une étape, préalable à l'étape pour lire la valeur courante dudit paramètre, pour déclencher l'acquisition de ladite valeur par lesdits moyens de capture et inscrire ladite valeur comme valeur courante.

Selon un deuxième objet, l'invention concerne un produit programme d'ordinateur comportant une ou plusieurs instructions de programme exploitables par une unité de traitement d'un module électronique, ladite unité de traitement coopérant avec des moyens de capture et des moyens de mémorisation, qui, lorsqu'elles sont exécutées ou interprétées par ladite unité de traitement, déclenchent la mise en œuvre d'un procédé maintenir un équilibre d'un paramètre physico-chimique d'un milieu conforme à l'invention.

Selon un troisième objet, l'invention concerne un module électronique comportant une unité de traitement coopérant avec des moyens de capture et des moyens de mémorisation. Avantageusement mais non limitativement, pour mettre en œuvre un procédé pour maintenir un équilibre d'un paramètre physico-chimique d'un milieu, ledit module électronique comporte dans lesdits moyens de mémorisation, des instructions d'un produit programme d'ordinateur conforme à l'invention.

D'autres caractéristiques et avantages apparaîtront plus clairement à la lecture de la description qui suit, se rapportant à un exemple de réalisation donné à titre indicatif et non limitatif, et à l'examen des figures qui l'accompagnent parmi lesquelles :
- la figure 1 décrit un exemple préféré mais non limitatif d'un système de régulation de paramètres physico-chimiques d'une eau de baignade d'une piscine ;
- la figure 2 décrit un schéma fonctionnel d'un système de régulation d'un milieu comportant un moyen de capture, des moyens de mémorisation et une unité de traitement pour mettre en œuvre un procédé pour maintenir en équilibre les paramètres physico-chimiques dudit milieu conforme à l'invention ;
- la figure 3 décrit un mode de réalisation préféré mais non limitatif d'un schéma fonctionnel d'un procédé pour maintenir un tel équilibre conforme à l'invention.

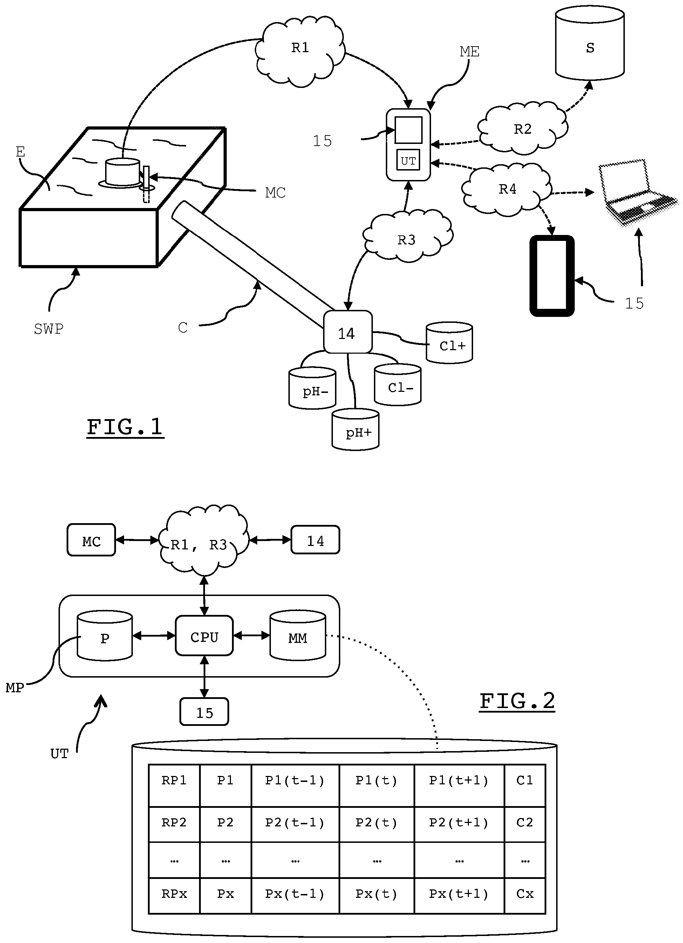

A titre d'exemple d'application préféré mais non limitatif, l'invention sera décrite au travers d'une application relative au maintien d'un équilibre d'un ou plusieurs paramètres de l'eau de baignade E d'une piscine SWP. Une telle application est notamment décrite au travers d'un système de régulation conforme à l'invention en lien avec la figure 1. Un tel système de régulation conforme à l'invention comprend des moyens de capture MC, inséré dans l'eau de baignade E de ladite piscine SWP. Un automate de distribution d'agents chimiques 14 dudit système de régulation est alors relié au bassin de ladite piscine SWP par un ou plusieurs conduits C, permettant d'y libérer un ou plusieurs agents chimiques, tel que du chlore par exemple. Pour cela, un tel automate 14 peut coopérer avec un ou plusieurs réservoirs d'agents chimiques pH-, pH+, Cl-, Cl+. Ledit système de régulation comporte alors une unité de traitement UT coopérant avec une interface homme-machine de consigne et de restitution 15. Ladite unité de traitement UT coopère en outre avec lesdits moyens de capture MC et ledit automate 14. Ces derniers peuvent être directement connectés par un bus filaire à ladite unité de traitement UT ou, en variante, être distants de ladite unité UT et coopérer via une liaison sans fil, par exemple au travers d'un réseau R1 ou R3 de type Internet, Intranet, Bluetooth ou WiFi. L'unité de traitement UT peut en outre coopérer avec un serveur S distant, via par exemple, une liaison sans fil R2, de type internet.

Selon une variante de réalisation avantageuse mais non limitative de l'invention décrite en lien avec la figure 1, l'interface homme-machine 15 et l'unité de traitement UT dudit système de régulation peuvent coopérer au sein d'un même équipement électronique ME. En variante, l'interface homme-machine 15 et l'unité de traitement UT peuvent être dissociées. Par exemple, l'interface homme-machine 15 peut être celle d'un dispositif électronique tiers, tel qu'un téléphone mobile ou un microordinateur, ladite interface 15 et ladite unité de traitement UT communiquant alorss via une liaison sans fil R4, par exemple Bluetooth, WIFI ou Ethernet.

En variante ou en complément, l'automate de distribution 14 peut être suppléé par une action humaine visant, par exemple, à actionner manuellement un filtre ou à introduire un galet de chlore dans l'eau de baignade de la piscine. Lorsque la distribution d'agents chimiques est effectuée manuellement, un utilisateur du système de régulation peut ainsi prendre connaissance de l'action à effectuer au travers d'une consigne restituée par l'interface homme-machine de restitution 15. Ladite consigne peut être préalablement élaborée par l'unité de traitement UT. Une fois l'action réalisée, l'utilisateur peut ensuite saisir, au travers d'une interface homme-machine de consigne 15, une consigne visant à acquitter ladite action, ladite consigne étant traitée par ladite unité de traitement UT.

La figure 2 présente l'architecture structurelle d'un système 10 de régulation d'une piscine conforme à l'invention. Pour maintenir en équilibre un ou plusieurs paramètres physico-chimiques de ladite piscine, un système de régulation conforme à l'invention comporte une unité de traitement UT, compris par exemple au sein d'un module électronique ME à la disposition de la personne chargée de l'entretien de la piscine, tel que décrit en lien avec la figure 1. Un tel module électronique ME peut consister en un appareil dédié ou consister en un ordinateur personnel ou un téléphone mobile ou tout autre équipement électronique. Une telle unité de traitement UT comporte avantageusement un microcontrôleur CPU coopérant, par couplage et/ou par bus filaire, avec des moyens de mémorisation de données MM et/ou de programmes MP. De tels moyens de mémorisation consistent essentiellement en une ou plusieurs mémoires non volatiles. Les moyens de mémorisation de programmes MP sont avantageusement agencés pour enregistrer notamment des instructions d'un programme P qui, lorsqu'elles sont exécutées ou interprétées par ledit microcontrôleur CPU, déclenchent la mise en oeuvre d'un procédé pour maintenir un équilibre d'un ou plusieurs paramètres physico-chimiques d'un milieu selon l'invention. Les moyens de mémorisation de données MM, quant à eux, sont notamment agencés pour enregistrer des structures de données nécessaires à la mise en oeuvre d'un procédé conforme à l'invention. A titre d'exemples non limitatifs, lesdites données peuvent être enregistrées dans des tables comprenant un ou plusieurs enregistrements ou des structures de données chaînées.

En variante ou en complément, toujours selon un mode de réalisation non limitatif d'un système de régulation conforme à l'invention, ce dernier peut comporter un ou plusieurs moyens de capture MC coopérant avec l'unité de traitement UT. Cette dernière est dès lors chargée de récolter les données fournies par lesdits moyens de capture MC. Les moyens de capture MC peuvent mesurer un ou plusieurs paramètres physico-chimiques en lien avec un milieu, tels qu'à titre d'exemples non limitatifs, le pH, le TAC, le TH, le potentiel d'oxydo-réduction et/ou la température. Comme l'indiquent les figures 1 et 2, les moyens de capture MC produisent dès lors une valeur, ci-après dénommée valeur courante Px(t), du paramètre Px considéré à chaque unité de temps t déterminée. La valeur courante Px(t) est alors enregistrée dans les moyens de mémorisation de données MM. Selon une variante de réalisation non limitative, l'enregistrement d'une donnée temporelle t caractérisant la période courante peut être conjointement réalisée avec celui de la valeur courante Px(t). A titre d'exemples non limitatifs, les moyens de capture MC peuvent consister en une sonde pH ou une sonde d'oxydo-réduction, respectivement dédiés à la mesure de la valeur courante du pH et du potentiel oxydant de l'eau E de la piscine SWP.

En variante ou en complément, l'unité de traitement UT peut en outre coopérer avec un actionneur 14, permettant de délivrer dans le milieu E une quantité donnée d'agent chimique. Selon la figure 1, un tel actionneur 14 peut, à titre d'exemples non limitatifs, consister en un ou plusieurs distributeurs automatiques de chlore Cl-, Cl+ et/ou d'agents chimiques basique pH+ ou acide pH-, et être par exemple directement connecté par un bus filaire à ladite unité de traitement UT, ou en variante être distant de ladite unité de traitement UT et coopérer via une liaison sans fil, par exemple au travers d'un réseau R1, R3 de type Internet, intranet, Bluetooth, WiFi, ou toute autre protocole équivalent.

En variante ou en complément, l'unité de traitement UT peut en outre coopérer avec une interface homme-machine de consigne et de restitution 15. Ladite interface 15 permet à un utilisateur du système de recevoir des recommandations d'actions correctives élaborées par l'unité de traitement UT, et d'émettre, par exemple, un message d'acquittement de l'accomplissement d'une telle action, si celle-ci requiert une intervention manuelle, tel que décrit précédemment au regard de la figure 1. Une telle interface de consigne et de restitution 15 peut, par exemple, consister en un écran tactile ou se présenter sous la forme de tout autre moyen de consigne et de restitution permettant à un utilisateur d'interagir avec ledit système de régulation. En variante, un système de régulation conforme à l'invention peut coopérer avec deux interfaces homme-machine 15 distinctes pour traduire des actions émanant d'un utilisateur et pour restituer à celui-ci des contenus graphiques et/ou sonores de manière dissociée. Une telle interface de consigne peut, par exemple, consister en un clavier, un microphone, une souris ou tout autre dispositif de pointage. De son côté, une telle interface de restitution peut consister, par exemple, en un écran ou un haut-parleur.

Pour faciliter la compréhension de l'invention, les moyens de mémorisation MP et MM sont représentés dans la figure 2 comme étant des éléments fonctionnels distincts. Les moyens de mémorisation MM et MP pourraient également et éventuellement ne constituer qu'une seule et même ressource physique. De la même manière, l'unité de traitement UT pourrait être celle d'un ou de plusieurs dispositifs électroniques communicants coopérant avec les moyens de mémorisation MM et MP. L'invention peut dès lors être adaptée à un environnement informatique de type client/serveur, c'est-à-dire que des dispositifs électroniques clients, tels qu'une unité de récolte des données issues des moyens de capture MC, peuvent se connecter à un serveur S mettant en œuvre un procédé pour maintenir un équilibre d'un ou plusieurs paramètres physico-chimiques d'un milieu conforme à l'invention, au travers d'un réseau de communication R2. Ainsi, les dispositifs électroniques peuvent accéder aux fonctionnalités offertes par ledit serveur S, tout en préservant les coûts de mise en œuvre.

Dans un exemple préféré mais non limitatif de l'invention, des données peuvent être enregistrées dans une ou plusieurs structures de données, comportant chacune un ou plusieurs enregistrements, au sein des moyens de mémorisation de données MM. A titre d'exemple non limitatif, lesdits enregistrements peuvent être respectivement dédiés ou associés à un ou plusieurs paramètres de milieu et/ou à une ou plusieurs actions correctives. Les différentes structures de données précédemment citées peuvent, en variante, ne constituer qu'une seule entité logique ou suivre tout autre mode de représentation et ne sauraient constituer une quelconque limitation pour l'invention.

La structure de données, comportant des enregistrements dédiés à un ou plusieurs paramètres de milieu, peut ainsi comporter des enregistrements RP1 ... RPx, respectivement associés à des paramètres de milieu P1 ... Px. Les enregistrements peuvent être regroupés sous la forme d'un tableau ordonné de x enregistrements. Ainsi, le premier enregistrement du tableau comporte des données relatives à un premier paramètre P1, le deuxième élément du tableau comporte des données relatives à un deuxième paramètre P2 et ainsi de suite, jusqu'au paramètre Px enregistrant les données relatives à un paramètre de rang x. Par mesure de simplification, nous nommerons « Px » l'un quelconque des paramètres de milieu P1, P2, ..., Px et « RPx » l'enregistrement qui lui est associé.

Chaque enregistrement RPx, associé à un paramètre physico-chimique Px d'un milieu E, peut être agencé pour mémoriser des données nécessaires et relatives au maintien de l'équilibre dudit paramètre Px. Lesdites données associées à un paramètre physico-chimique Px déterminé peuvent dès lors être relatives à une période courante t, et correspondre, à titre d'exemples, à un identifiant de paramètre Px, une valeur courante Px(t) mesurée ou calculée dudit paramètre Px respectivement par les moyens de capture MC ou par l'unité de traitement UT, une valeur Px(t-n) acquise et mémorisée lors d'une période précédente t-n, une valeur nominale Px(t+n) estimée du paramètre Px pour une période subséquente t+n, n étant un entier supérieur ou égal à 1, une valeur d'échéance Cx pour recouvrer une telle valeur nominale Px(t+n) de paramètre et éventuellement la valeur d'une distance Δ entre ladite valeur nominale Px(t+n) et la valeur courante Px(t). Au sens de l'invention et dans tout le document, on entend par « valeur nominale », une valeur cible ou attendue décrivant un état normal du milieu. Par exemple, dans le cadre d'une piscine, la valeur nominale du paramètre pH de l'eau peut être fixée à 6,8. En complément, un tel enregistrement peut mémoriser une valeur caractérisant l'horodatage de la période courante t et des données d'anticipation DA. De telles données d'anticipation DA peuvent, à titre d'exemples non limitatifs, consister en des données de prévision météorologique ou encore en des données caractérisant la fréquentation humaine ou animale future du milieu, etc.

En outre, un tel enregistrement RPx, associé à un paramètre physico-chimique Px d'un milieu E, peut être agencé pour mémoriser des données nécessaires à l'élaboration d'une action au regard d'une distance Δ préalablement calculée. Un tel enregistrement peut comporter un identifiant d'action, des données produits caractérisant, à titre d'exemples non limitatifs, des spécifications relatives à un dosage d'un agent chimique déterminé, un attribut caractérisant une priorité d'exécution, des caractéristiques du milieu tel qu'à titre d'exemple, le type de revêtement du bassin de la piscine, etc. Les moyens de mémorisation de programmes MP peuvent, à ce titre, comporter une banque d'actions correctives distinctes, éventuellement ajustée par un ou plusieurs paramètres d'exécution de l'action, dont les valeurs par défaut respectives peuvent être mémorisées dans une table dans les moyens de mémorisation de données MM.

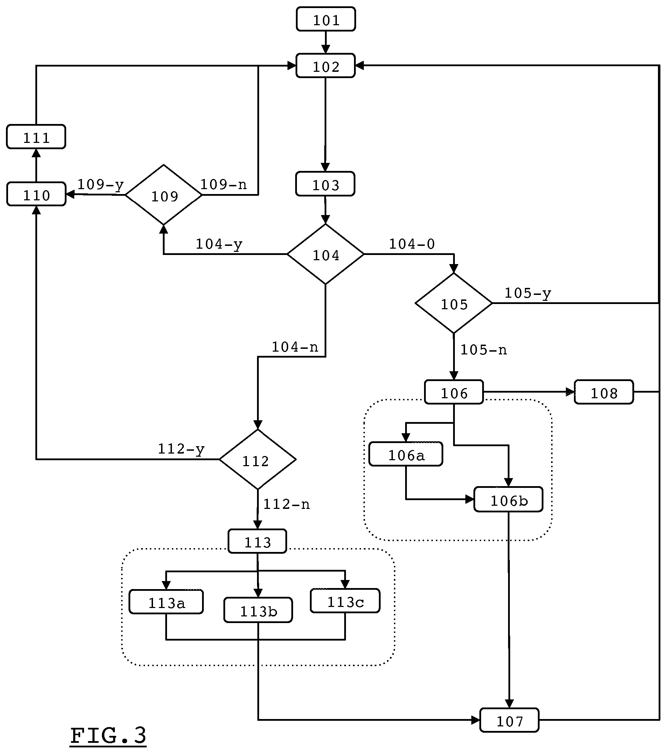

Décrivons à présent, en lien avec la figure 3 ; un schéma fonctionnel d'un procédé 100 pour maintenir en équilibre un ou plusieurs paramètres physico-chimiques d'un milieu E conforme à l'invention, tel que par exemple, une piscine ou le sol d'un jardin.

Un procédé 100 pour maintenir en équilibre un ou plusieurs paramètres physico-chimiques d'un milieu E conforme à l'invention peut être mis en œuvre itérativement selon une période donnée, telle qu'à titre d'exemples non limitatifs, toutes les six heures ou toutes les vingt-quatre heures ou selon toute autre période prédéterminée. La mise en œuvre dudit procédé 100 peut, en variante ou en complément, être déclenchée en réaction à un évènement déclencheur, tel qu'à titre d'exemples non limitatifs, une mise à jour des moyens de mémorisation de données MM ou une nouvelle inscription dans lesdits moyens de mémorisation de données MM. Selon un autre exemple d'évènement déclencheur, ledit procédé peut être mis en œuvre à la suite d'une sollicitation d'un utilisateur du système de régulation, c'est-à-dire suite à la réception d'un message de consigne émanant de l'interface homme-machine de consigne 15 dudit système de régulation.

En outre, le procédé 100 peut être mis en œuvre pour maintenir ou réguler une pluralité de paramètres physico-chimiques d'un milieu E successivement ou conjointement. Eventuellement, chaque paramètre physico-chimique Px peut avoir une périodicité de régulation et/ou de suivi identique ou différente des autres. A titre d'exemple préféré mais non limitatif, le procédé 100 mis en œuvre pour maintenir ou réguler le pH peut être mis en œuvre une fois par jour, alors que le procédé 100 mis en œuvre pour réguler le Titre Alcalimétrique Complet TAC peut être déclenché une fois par semaine.

En liaison avec la figure 3, un procédé 100 pour maintenir un paramètre physico-chimique d'un milieu E conforme à l'invention, avantageusement mis en œuvre par l'unité de traitement UT d'un système de régulation d'un milieu, tel que celui décrit en lien avec les figures 1 et 2, comporte une première étape 102 et une deuxième étape 103 pour respectivement lire la valeur courante Px(t) et la valeur nominale Px(t+n) d'un enregistrement RPx associé à un paramètre Px au sein des moyens de mémorisation de données MM, tel qu'à titre d'exemple, un enregistrement dédié à la régulation du pH. Une telle valeur nominale Px(t+n) peut, à titre d'exemple non limitatif, consister en une valeur déterminée selon laquelle le milieu est équilibré et sain pour être exploité correctement. Comme précédemment mentionné, le pH de l'eau d'une piscine peut idéalement être compris entre 6,9 et 7,6. En dehors de cette plage de valeurs, il peut être nécessaire de réguler la valeur du pH en introduisant un agent chimique dans l'eau de baignade de ladite piscine, par exemple, un agent chimique basique lorsque le pH est trop acide et un agent chimique acide lorsque le pH est trop basique. Ainsi, la valeur nominale Px(t+n) peut alors être égale à tout valeur comprise entre 6,9 et 7,6. En variante ou un complément, l'enregistrement RPx dédié ou associé à un paramètre Px d'un milieu peut comporter une valeur nominale minimale, par exemple pour un enregistrement dédié au pH une valeur nominale minimale égale à 6,9, et une valeur nominale maximale, par exemple pour un enregistrement dédié au pH une valeur nominale minimale égale à 7,6. L'étape 103 consiste dès lors à lire lesdites valeur nominale maximale et valeur nominale minimale. Enfin, toujours en variante ou en complément, la valeur nominale Px(t+n) d'un paramètre de milieu Px peut consister en un intervalle de valeurs.

Comme nous le verrons dans la suite du document, la valeur courante Px(t) d'un paramètre Px, lue à l'étape 102, peut être avantageusement et préalablement estimée ou mesurée. Lorsqu'une telle valeur courante Px(t) est mesurée, ledit procédé 100 peut comporter une étape 101, préalable à l'étape 102 pour lire la valeur courante Px(t), pour déclencher l'acquisition de ladite valeur Px(t) par les moyens de capture MC d'un système de régulation, tel que celui décrit en lien avec les figures 1 et 2 et inscrire ladite valeur comme valeur courante Px(t) au sein des moyens de mémorisation de données MM. Lorsque le procédé 100 est agencé pour maintenir le pH de l'eau d'une piscine, de tels moyens de capture MC peuvent consister en une sonde pH, permettant de mesurer la valeur courante Px(t) du pH dans l'eau de piscine lors de la période t.

Un procédé 100 pour maintenir un paramètre physico-chimique d'un milieu conforme à l'invention comporte ensuite une étape 104 pour lire la valeur contenue dans un champ de l'enregistrement RPx dédié au paramètre Px de milieu mémorisant une éventuelle valeur d'échéance Cx.

Selon une première situation symbolisée par le lien 104-0 en figure 3, un tel champ peut comporter une valeur prédéterminée caractérisant une absence de définition d'échéance. Une telle valeur peut dès lors, à titre d'exemple non limitatif, consister en une valeur « vide » ou « nulle », connue généralement en langage informatique sous la dénomination « VOID ». Lorsqu'aucune définition d'échéance n'a été formulée, le procédé 100 comporte alors une étape 105 pour estimer une distance Δ entre la valeur nominale Px(t+n) et la valeur courante Px(t). Une telle distance Δ permet d'attester une relative égalité, situation symbolisée par le lien 105-y en figure 3, ou au contraire, une claire disparité, situation symbolisée par le lien 105-n en figure 3, entre lesdites valeurs nominale et courante. Si ladite distance atteste d'une relative égalité, la courante itération du procédé 100 s'achève : le procédé sera à nouveau mis en œuvre à la prochaine itération.

A titre d'exemple non limitatif, lorsque le paramètre étudié Px est le pH, l'étape 105 peut consister à soustraire la valeur courante Px(t), par exemple la valeur 4,5, à la valeur nominale Px(t+n), par exemple 7,3. Si le résultat ainsi obtenu est positif, le pH de la piscine peut être considéré trop acide. Au contraire, si le résultat est négatif, le pH de la piscine peut être considéré trop basique. Enfin, lorsque le résultat est égal à zéro « 0 », la valeur courante Px(t) et la valeur nominale Px(t+n) sont sensiblement identiques, c'est-à-dire que le pH de la piscine a atteint ou est égal à sa valeur nominale ou valeur cible, l'équilibre du pH au sein de la piscine est donc maintenu. En variante ou en complément, il est possible de déterminer une plage de tolérance, par exemple +0,5 et -0,5, autour de la valeur de la distance Δ pour lequel la valeur courante Px(t) est sensiblement égale à la valeur nominale Px(t+n), par exemple zéro. Si le résultat de ladite soustraction est compris dans ladite plage de tolérance, le procédé 100 peut, lors de l'étape 105, estimer que la valeur nominale Px(t+n) et la valeur courante Px(t) sont identiques. Au contraire lorsque la valeur de la distance Δ est différente de zéro et/ou que ladite valeur de la distance Δ n'est pas comprise dans une plage de tolérance définie, le procédé 100 peut, lors de l'étape 105, estimer que la valeur nominale Px(t+n) et la valeur courante Px(t) sont distinctes. En variante ou en complément, l'étape 105 dudit procédé 100 peut consister à comparer la valeur courante Px(t) à la valeur nominale Px(t+n) du paramètre physico-chimique d'intérêt Px selon des fonctions mathématiques connues pour déterminer une distance Δ entre la valeur courante Px(t) et la valeur nominale Px(t+n) dudit paramètre Px. A titre d'exemple non limitatif, l'étape 105 peut consister à estimer la distance Δ sous la forme d'un ratio entre la valeur courante Px(t) et la valeur nominale Px(t+n), par exemple en divisant la valeur courante Px(t) par la valeur nominale Px(t+n).

Si l'étape 105 atteste d'une disparité entre la valeur courante Px(t) et la valeur nominale Px(t+n), situation symbolisée par le lien 105-n en figure 3, alors un procédé 100 pour maintenir un paramètre Px d'un milieu comporte une étape 106 pour élaborer une action corrective pour tenter de recouvrer une valeur courante Px(t) proche de la valeur nominale Px(t+n). Une telle étape 106 peut consister à élaborer une action en fonction de la distance Δ préalablement estimée. A titre d'exemple non limitatif, si la distance Δ traduit un pH trop acide, l'étape 106 peut consister à élaborer une action visant à augmenter la valeur du pH, par exemple en ajoutant un agent chimique basique pH+. La quantité d'agent chimique à introduire dans le milieu, plus particulièrement une piscine, est généralement déterminée par le fabricant dudit agent chimique et dépend du volume d'eau de baignade et de la valeur du pH dans la piscine. Afin d'éviter un ajout excessif de produit, l'étape 106 peut consister à extraire des données fabricants préalablement enregistrées dans les moyens de mémorisation de données MM et calculer une quantité ou un dosage d'agent chimique idoine. La quantité d'agent chimique à introduire dans ladite piscine peut être pondérée par la distance Δ entre la valeur courante Px(t) et la valeur normale Px(t+n). En variante ou en complément, une telle étape 106 pour élaborer une action corrective peut en outre consister à estimer un temps de filtration de l'eau de baignade E de la piscine SWP, telle que décrite en lien avec la figure 1, pendant une durée déterminée, par exemple trois heures.

Selon un mode de réalisation non limitatif d'un procédé pour maintenir un paramètre physico-chimique Px conforme à l'invention, l'étape 106 peut comporter une étape 106a pour estimer une valeur nominale Px(t+n) au regard de l'action élaborée. Une telle étape 106a peut, par exemple, consister à estimer l'effet d'un apport d'une quantité d'agent chimique, préalablement calculée, sur la valeur courante Px(t) du paramètre de milieu Px considéré lors d'une période suivante t+n. Ainsi, si la valeur courante Px(t) du pH est égale à 4,2, l'ajout d'une quantité déterminée d'un agent chimique basique devrait faire tendre la valeur du pH vers 6,9. La valeur courante Px(t) devrait donc tendre vers une valeur nominale Px(t+n) égale à 6,9.

Selon un autre mode de réalisation non limitatif d'un procédé pour maintenir un paramètre physico-chimique Px conforme à l'invention, en variante ou un complément, l'étape 106 peut comporter une étape 106b pour définir ou estimer une valeur d'échéance Cx, c'est-à-dire une date à laquelle ou une durée au bout de laquelle la valeur courante Px(t) et la valeur nominale Px(t+n) du paramètre Px devraient être sensiblement identiques. Une valeur d'échéance Cx peut ainsi consister en une valeur prédéterminée, caractérisant la durée ou le délai estimé pour que la valeur courante Px(t) d'un paramètre Px considéré recouvre une valeur nominale Px(t+n). Lorsque le paramètre considéré Px est le pH, après l'ajout d'un agent chimique, il peut être nécessaire d'attendre une heure pour que le pH au sein de l'eau d'une piscine se stabilise. Pour ce faire, la valeur d'échéance Cx peut être associée à un compteur croissant ou décroissant permettant de comptabiliser un nombre d'unités de temps déterminé, ledit compteur étant mouvementé régulièrement à chaque unité de temps. Toute autre méthode ou tout autre élément matériel pourrait être utilisée pour estimer ou définir une telle valeur d'échéance. Ainsi, en variante ou en complément, selon l'invention, la valeur d'échéance Cx pourrait consister en une datation absolue, c'est-à-dire une date et une heure. Pour ce faire, l'unité de traitement UT d'un système de régulation comporte ou coopère avec une horloge interne, permettant ainsi de comparer la date et l'heure courante à ladite valeur d'échéance.

Un procédé 100 pour maintenir un paramètre Px d'un milieu comporte à présent une étape 107 pour déclencher une action corrective. Une telle étape 107 peut consister à mettre à jour l'enregistrement dédié au paramètre Px dont la valeur courante a été à l'initiative de l'élaboration de ladite action, par exemple en y inscrivant les valeurs respectives précédemment estimées d'échéance Cx et normale Px(t+n). Lorsque l'unité de traitement UT d'un système de régulation, tel que décrit en lien avec la figure 2, coopère avec un actionneur 14, l'étape 107 peut, en variante ou en complément, consister à élaborer une commande à destination dudit actionneur 14, ladite commande comportant la nature et la quantité d'agent chimique à apporter à l'eau de la piscine. Egalement, ladite commande peut comporter un attribut caractérisant la mise en œuvre de l'actionneur 14 coopérant avec l'unité de traitement UT.

Selon une autre variante de réalisation d'un procédé 100 pour maintenir un paramètre Px d'un milieu conforme à l'invention, lorsque l'unité de traitement UT coopère avec une interface homme-machine de restitution 15, l'étape 107 peut consister à élaborer et transmettre une consigne sous la forme d'un message à destination de ladite interface 15 comportant des données relatives à une action déterminée, précédemment élaborée et estimée à l'étape 106. Ladite action devra être accomplie par le propriétaire, l'opérateur ou plus généralement l'utilisateur du milieu. Une fois le message décodé et restitué par ladite interface 15, ledit utilisateur est ainsi en mesure de prendre connaissance de l'action corrective recommandée et peut l'effectuer par lui-même.

L'évolution d'un premier paramètre P1 d'un milieu peut être liée à celle d'un deuxième paramètre P2 du même milieu. Ainsi, il peut donc être nécessaire de surveiller l'évolution conjointe d'une pluralité de paramètres P1 et P2 lors du déclenchement d'une action corrective, par exemple en estimant l'impact d'une action corrective élaborée pour un premier paramètre P1 sur la valeur nominale P2(t+n) d'un deuxième paramètre P2. Le procédé 100 peut dès lors, en variante ou en complément, comporter une étape 108 pour estimer une valeur nominale P2(t+n) d'un deuxième paramètre P2, selon une action précédemment élaborée pour le premier paramètre P1 lors de l'étape 106. A titre d'exemple non limitatif, lors d'une variation de la valeur du premier paramètre lié au pH, le deuxième paramètre lié au potentiel d'oxydoréduction d'une piscine peut devenir instable. Pour anticiper cette instabilité, il peut être nécessaire d'estimer une valeur nominale P2(t+n) du paramètre P2 lié au potentiel d'oxydo-réduction au regard de l'action corrective élaborée pour le premier paramètre P1 lié au pH. Cette anticipation d'effet sur tout ou partie des paramètres du milieu peut permettre d'éviter de déclencher des actions correctives inefficaces et/ou improductives, voire même perturbantes, pour l'équilibre du milieu.

Une telle étape 108 peut en outre consister à estimer et mettre à jour la valeur courante P2(t) d'un deuxième paramètre du milieu P2. En effet, certaines valeurs courantes Px(t) de paramètres Px du milieu peuvent être estimées. C'est, par exemple, le cas de la valeur du stabilisateur du chlore connu sous le nom d'acide iso-cyanurique généralement non mesurable par des moyens de capture MC conventionnels. A titre d'exemple non limitatif, une action corrective visant à augmenter la valeur du potentiel d'oxydo-réduction, peut consister à ajouter une quantité estimée de chlore. Un tel agent chimique est généralement introduit conjointement avec une quantité déterminée d'acide iso-cyanurique. L'apport de chlore peut donc faire varier la valeur courante P2(t) du paramètre P2 lié à l'acide iso-cyanurique de manière déterministe.

Selon une deuxième situation symbolisée par le lien 104-n en figure 3, la valeur du champ pour mémoriser une valeur d'échéance Cx d'un paramètre Px peut être une valeur prédéterminée caractérisant une fin de période de recouvrement. Plus précisément, une telle valeur signifie que le délai d'attente estimé, pour qu'une action corrective produise ses effets, est atteint. Comme évoqué précédemment, une telle deuxième situation peut découler d'une valeur de compteur Cx nulle ou, dans le cas où la valeur d'échéance Cx comporte une datation absolue, ladite valeur d'échéance est inférieure à la date courante.

Le procédé 100 comporte alors une étape 112, identique à l'étape 105 préalablement décrite, pour estimer une distance Δ entre la valeur nominale Px(t+n) et la valeur courante Px(t). Si ladite étape 112 atteste d'une distance Δ sensiblement nulle, c'est-à-dire que la valeur courante Px(t) du paramètre Px a recouvré une sensible normalité, alors ledit procédé 100 comporte une étape 110 pour acquitter l'échéance Cx d'une telle action corrective. A titre d'exemple non limitatif, une telle étape 110 peut consister à mettre à jour l'enregistrement associé au paramètre Px et y inscrire dans le champ pour mémoriser une valeur d'échéance Cx, la valeur prédéterminée caractérisant une absence de définition d'échéance. Éventuellement, l'étape 110 peut être mise en œuvre systématiquement lorsque la distance Δ est nulle.

Au contraire, si ladite étape 112 atteste d'une distance Δ non-nulle, c'est-à-dire que la valeur courante Px(t) demeure distincte ou éloignée de la valeur nominale Px(t+n), alors ledit procédé 100 comporte une étape 113 de correction de l'action corrective préalablement élaborée et déclenchée respectivement lors de l'étape 106 et 107, ladite action corrective étant considérée comme n'ayant pas produit les effets attendus. Pour corriger une telle action corrective, l'étape 113 peut comporter une étape 113a pour estimer une nouvelle valeur nominale Px(t+n) selon l'une des méthodes évoquées précédemment. En variante ou en complément, ladite étape 113 peut comporter une étape 113b pour définir une nouvelle valeur d'échéance Cx selon l'une des méthodes évoquées précédemment. Selon cette variante, l'unité de traitement UT accorde un délai supplémentaire pour que le paramètre recouvre sa normalité sans déclencher une nouvelle action corrective. En variante ou complément, l'étape 113 peut comporter une étape 113c consistant à élaborer une action corrective tierce, différente de celle élaborée et déclenchée à une période précédente. A titre d'exemple non limitatif, une telle action corrective tierce peut consister à vérifier la valeur courante d'un deuxième paramètre P2, par exemple la valeur courante P2(t) correspondant, à titre d'exemple non limitatif, à la valeur courante du TAC. En effet, lorsque la valeur du TAC est inférieure à quatre-vingt (80) milligrammes par litre, le pH peut devenir instable. Il est dès lors nécessaire d'augmenter la concentration du TAC dans l'eau d'une piscine par l'ajout d'un agent chimique adapté. Une action corrective tierce peut en outre consister à élaborer une consigne à destination d'un utilisateur du système et de déclencher la restitution de celle-ci par l'interface homme-machine de restitution 15. Une telle consigne peut, à titre d'exemple, consister à inciter l'utilisateur à aller vérifier l'état des équipements de sa piscine, par exemple l'état de fonctionnement d'un automate de filtration.

Comme d'ores et déjà mentionné, l'unité de traitement UT d'un système de régulation conforme à l'invention peut également coopérer avec une structure de données associée à différentes actions correctives, ladite structure d'actions comportant un ou plusieurs enregistrements associés à une ou plusieurs actions correctives, non représentée dans les figures à des fins de simplification. Chaque enregistrement peut en outre comporter un identifiant d'action, un identifiant de paramètre de milieu Px, et/ou un attribut caractérisant une priorité d'exécution. Ainsi, l'étape 106 pour élaborer une action corrective en lien avec un paramètre de milieu Px peut consister à rechercher dans ladite structure d'actions, un enregistrement dédié à une action corrective de priorité élevée, caractérisée, par exemple, par une valeur telle que la valeur entière « 1 » et associée au paramètre Px. Lors de la mise en œuvre de l'étape 113 de correction d'une action corrective, ladite étape 113 peut consister à rechercher un enregistrement associé à une action corrective de priorité moindre, caractérisée par exemple par une valeur entière supérieure comme la valeur « 2 » et associée au paramètre Px. Les actions correctives ainsi élaborées lors de l'étape 113 peuvent être déclenchées lors de l'étape 107 pour déclencher une action corrective précédemment décrite.

En variante ou en complément, un procédé 100 conforme à l'invention peut comporter une étape, non représentée en figure 3 à des fins de simplification, préalable à l'étape 113 pour élaborer une correction d'une action, pour restituer au moyen de l'interface homme-machine 15, un message de confirmation à destination de l'utilisateur ou de l'opérateur du milieu, ledit message étant élaboré, par l'unité de traitement UT, pour s'assurer de l'accomplissement de l'action recommandée à l'utilisateur lors de l'étape 107. L'étape 113 pour élaborer une correction d'action est mise en œuvre si et seulement l'unité de traitement UT décode préalablement un message d'acquittement, émis par l'interface homme-machine 15, comportant un attribut caractérisant un accomplissement d'action en réaction ou en réponse à l'interprétation dudit message de confirmation par l'utilisateur.

En variante ou en complément, un procédé 100 conforme à l'invention peut comporter une étape 111, postérieure à l'étape 110 pour acquitter l'échéance Cx d'une action relative à un paramètre Px, pour estimer la valeur nominale P2(t+n) et/ou la valeur courante P2(t) d'un deuxième paramètre P2 à partir de l'action corrective acquittée lors de l'étape 110. A titre d'exemple non limitatif, le milieu peut avoir recouvré un équilibre à la suite d'un ajout d'un agent chimique désinfectant, tel qu'à titre d'exemple du chlore, un tel ajout étant lié au potentiel d'oxydo-réduction. La valeur courante P2(t) d'un deuxième paramètre P2, par exemple l'acide iso-cyanurique, doit alors être augmentée d'une unité. En effet, la concentration en acide iso-cyanurique, consistant un stabilisateur de chlore, ne peut être mesurée par des instruments de mesure. Elle doit être estimée lors d'un apport d'un galet de chlore dans un skimmer par exemple.

Selon une troisième situation symbolisée par le lien 104-y en figure 3, la valeur du champ pour mémoriser une valeur d'échéance Cx peut consister en une valeur, autre qu'une valeur prédéterminée caractérisant une absence de définition d'échéance ou une fin de période de recouvrement. Ainsi, le procédé 100 peut comporter une étape 109, identique à l'étape 105 préalablement décrite, pour estimer une distance Δ entre la valeur nominale Px(t+n) et la valeur courante Px(t) d'un paramètre Px. Si ladite étape 109 atteste d'une distance Δ sensiblement nulle, c'est-à-dire que le paramètre concerné a recouvré sa normalité plus rapidement qu'escompté, l'unité de traitement UT peut mettre en œuvre l'étape 110 pour acquitter l'échéance Cx. Dans le cas contraire, la courante itération du procédé 100 s'achève : le procédé 100 sera à nouveau mis en œuvre à la prochaine itération.

Nous pouvons ainsi remarquer que, contrairement à un système selon l'art antérieur, l'invention permet de ne pas entreprendre immédiatement une nouvelle action corrective, pour tenter de corriger le paramètre physico-chimique considéré ou plus généralement le milieu, en adjoignant par exemple un agent chimique actif. Bien au contraire, alors qu'un procédé conforme à l'invention détecte une valeur Px(t) « anormale » d'un paramètre physico-chimique Px, ledit procédé accorde, selon une métaphore bien connue, du temps au temps, de sorte que l'action corrective précédemment engagée, pour laquelle une valeur d'échéance Cx a été initialisée, ladite échéance traduisant un délai d'action, puisse produire son effet. Ainsi, l'invention permet d'adjoindre ou d'introduire un minimum d'agents chimiques correctifs, en tenant compte d'un temps nécessaire à l'accomplissement d'une action corrective. Prenons pour illustrer cet avantage, l'exemple d'un bassin d'une piscine dont le revêtement est par nature intrinsèquement basique, c'est-à-dire entrainant inexorablement par sa structure et/ou sa composition, à son simple contact, un pH de l'eau dudit bassin supérieur à 7. Alors qu'une action corrective visant à ajouter un agent chimique acide tel que l'acide chlorhydrique ou un agent dit «pH-» produit classiquement, dans un bassin doté d'un revêtement différent, un effet quasi-immédiat, ladite action corrective peut demander un délai supplémentaire dans le bassin concerné par ledit revêtement basique. Un système connu aurait tendance à requérir l'ajout ou ajouter directement continuellement un tel acide tant que le paramètre Px, en l'espèce le pH, n'a pas recouvré une valeur proche de 6,9, quitte à ce que cet apport excessif et continu d'agent induise, à son tour, un déséquilibre entraînant un apport d'un agent à présent basique pour corriger un pH trop faible et ainsi de suite, tel un yoyo ou un jouet en déséquilibre permanent. Les système et procédé selon l'invention n'ont pas recours à un tel apport complémentaire d'acide chlorhydrique, mais laisse ce dernier agir le temps nécessaire et déterminé par l'échéance Cx, minimisant ainsi l'apport d'agents chimiques correctifs.

Selon une variante ou un complément, l'étape 102 pour lire la valeur courante Px(t) d'un paramètre physico-chimique Px, peut en outre consister à lire une valeur courante précédentes Px(t-n) et calculer une nouvelle valeur courante à partir desdites valeurs lues. Cette variante peut permettre de limiter une trop grande réactivité ou variabilité d'un paramètre, voire encore d'anticiper un impact futur de l'environnement sur le milieu, en altérant volontairement la valeur courante dudit paramètre. Selon un exemple de réalisation, un tel calcul préalable peut consister à calculer une moyenne entre la valeur courante Px(t) et les valeurs courantes dudit paramètre Px d'une ou plusieurs périodes écoulées t-n. L'étape 102 peut consister dès lors à lire et extraire les valeurs respectives desdites périodes écoulées, puis de calculer la moyenne desdites valeurs selon des fonctions informatiques connues.

En variante ou en complément, l'étape 102 pour lire la valeur courante Px(t) d'un paramètre physico-chimique Px peut en outre consister à rechercher et lire dans les moyens de mémorisation de données MM, une ou plusieurs données d'anticipation et calculer, selon des méthodes mathématiques connues, une nouvelle valeur courante du paramètre Px à partir desdites valeurs de données d'anticipation lues.

Les données d'anticipation peuvent, lorsqu'elles sont combinées à la valeur courante Px(t), par des méthodes de calcul connues, permettre d'anticiper des évènements ou perturbations pouvant rompre l'équilibre entre des paramètres d'un milieu. L'objectif d'une telle anticipation est de déclencher une action corrective, en prévision d'un déséquilibre futur, par exemple en prévision d'une fréquentation accrue d'un bassin d'une piscine, afin de sensiblement conserver ou recouvrer un équilibre plus rapidement à l'issue de ladite perturbation. Une telle action peut être assimilée à un traitement préventif de l'eau de la piscine en anticipant une telle perturbation. Une telle action d'anticipation peut ainsi consister à apporter un agent chimique désinfectant à faible concentration avant l'occurrence de la perturbation, afin notamment de préparer le milieu à une telle perturbation, en administrant un traitement de moindre quantité d'agent chimique par rapport au traitement correctif sans anticipation après l'occurrence de la perturbation. De la même manière, il est possible de minimiser ou simplifier des éventuelles actions correctives automatiques ou manuelles, liées à une telle perturbation grâce à une telle anticipation. A titre d'exemples non limitatifs, une pluie acide peut faire diminuer la valeur du pH d'une piscine. L'apport anticipatif d'agent chimique basique à faible dose, avant l'occurrence prochaine d'une telle pluie, permettra d'atténuer voire d'éviter toute perte d'équilibre. Au même titre, selon un autre exemple, une hausse de la fréquentation d'une piscine peut faire diminuer le potentiel d'oxydo-réduction. Un ajout anticipatif d'un agent chimique pertinent permettra de rehausser la valeur dudit potentiel d'oxydo-réduction et ainsi de garantir un pouvoir désinfectant de l'eau optimal lors de la hausse de la fréquentation.

Selon une autre variante de réalisation d'un procédé conforme l'invention, ce dernier peut comporter une étape pour calculer la valeur courante P3(t) d'un paramètre physico-chimique P3 synthétisé ou calculé à partir des valeurs courantes mesurables et/ou calculables d'autres paramètres P1 et P2. Ainsi, l'étape 102 peut consister à extraire des première et deuxième valeurs courantes P1(t) et P2(t) respectives de premier et deuxième enregistrements respectivement dédiés ou associés à des premier et deuxième paramètres P1 et P2 du milieu et estimer une troisième valeur courante P3(t) d'un troisième paramètre P3 à partir desdites première et deuxième valeurs courantes P1(t) et P2(t) et inscrire ladite troisième valeur courante P3(t) ainsi estimée dans ledit troisième enregistrement. Dès, les moyens de mémorisation MM peuvent comporter un enregistrement RP3 dédié ou associé audit troisième paramètre P3 du milieu, correspondant par exemple à l'indice de langelier IS. La valeur courante P3(t) peut ainsi être estimée ou calculée à partir de la valeur courante du pH, de la valeur des solides dissous TDS, de l'alcalinité de l'eau TAC, et du titre Hydrotimétrique TH, et de la température T de l'eau. Ladite étape 102 consiste ainsi à lire et extraire les valeurs courantes desdits paramètres nécessaires à un tel calcul, puis à produire ladite valeur courante selon un traitement donné, par exemple selon l'équation : IS= pH+T+TH+TAC-TDS. Ladite valeur produite peut être ensuite inscrite comme valeur courante P3(t) dudit paramètre P3.

En variante ou en complément, un procédé 100 conforme à l'invention peut en outre comporter une étape, non représentée sur les figures, pour initialiser ou mettre à jour la teneur des moyens de mémorisation MM et/ou MP. Une telle initialisation peut consister à affecter aux valeurs nominales Px(t+n) des valeurs issues de statistiques ou de moyennes élaborées à partir d'une banque de données recueillies par des moyens de capture MC de plusieurs systèmes de régulation conformes à l'invention. Une telle collecte et un tel croisement de valeurs issues d'une pluralité de milieux similaires au milieu considéré permettent d'estimer des comportements généraux desdits milieux. La structure associée aux actions correctives peut en outre être initialisée ou mise à jour périodiquement à partir de valeurs statistiques et/ou d'actions élaborées à partir d'une pluralité de données issues de milieux similaires. Le procédé 100 peut dès lors comporter une étape, non représentée sur les figures, pour exporter de telles données et pour constituer ou enrichir un telle banque de données.

L'invention a principalement été décrite en liaison avec le maintien d'un paramètre physico-chimique d'un milieu spécifique, plus particulièrement le pH de l'eau d'une piscine. En variante ou en complément, un procédé 100 conforme à l'invention peut être appliqué à tout autre paramètre du milieu considéré, tel que par exemple, le potentiel d'oxydo-réduction, le TAC, le TH, le TDS, ou tout autre paramètre mesurable dans le milieu.

En outre, l'invention a été décrite lors de son utilisation en lien avec la gestion d'une piscine. Elle peut également être mis en œuvre pour agir sur tout autre milieu, tel qu'à titre d'exemple lors de la gestion de l'équilibre entre des paramètres issus dans la terre, dans un vivarium, dans un aquarium etc.

## Revendications

1. Procédé (100) pour maintenir un équilibre d'un paramètre physico-chimique (P1,...,Px) d'un milieu (E), ledit procédé (100) étant mis en œuvre par une unité de traitement (UT) d'un système de régulation dudit milieu, ladite unité de traitement (UT) coopérant avec des moyens de mémorisation (MM) enregistrant des données en lien avec un paramètre de milieu (P1,...,Px), lesdits moyens de mémorisation (MM) comportant :
- un enregistrement (RP1,...,RPx) dédié audit paramètre de milieu (P1,...,Px) et agencé pour mémoriser une valeur courante (P1(t),...,Px(t)) dudit paramètre (P1,...,Px) et une valeur nominale (P1(t+n),...,Px(t+n)) dudit paramètre ;
ledit procédé (100) comportant :
- une étape (102) pour lire la valeur courante (P1(t),...,Px(t)) dudit paramètre (P1,...,Px) ; la valeur courante Px(t) étant la valeur mesurée ou calculée dudit paramètre Px respectivement par des moyens de capture MC ou par l'unité de traitement UT ;
- une étape (103) pour lire la valeur nominale (P1(t+n),...,Px(t+n)) dudit paramètre (P1,...,Px) ; la valeur nominale étant une valeur cible ou attendue décrivant un état normal du milieu ;
- une étape (105) pour estimer une distance (Δ) entre ladite valeur nominale (P1(t+n),..., Px(t+n)) et la valeur courante (P1(t),...,Px(t));
- une étape (107) pour déclencher une action selon la valeur de ladite distance (Δ) ;
ledit procédé (100) étant **caractérisé en ce que** :
- l'enregistrement dédié audit paramètre de milieu (P1,...,Px) comporte un champ pour mémoriser une date d'échéance (C1,...,Cx) pour que la valeur courante recouvre la valeur nominale ;
- ledit procédé (100) comporte :
∘ une étape (104) préalable à l'étape (107) pour déclencher une action, pour lire la valeur du champ pour mémoriser la date d'échéance (C1,...,Cx) ;
∘ l'étape (107) pour déclencher une action selon ladite distance (Δ) étant mise en œuvre si et seulement si :
▪ la valeur du champ pour mémoriser la date d'échéance (C1,...,Cx) consiste en une valeur prédéterminée caractérisant l'absence de définition de la date d'échéance (104-0) pour que la valeur courante recouvre la valeur nominale.

2. Procédé (100) selon la revendication précédente, comportant une étape (106), antérieure à l'étape (107) pour déclencher une action, pour élaborer ladite action selon la valeur de ladite distance (Δ).

3. Procédé (100) selon la revendication 2, pour lequel l'étape (106) pour élaborer une action comporte une étape (106a) pour estimer une valeur nominale (P1(t+n),...,Px(t+n)) dudit paramètre (P1,...,Px).

4. Procédé (100) selon la revendication 2 ou 3, pour lequel l'étape (106) pour élaborer une action comporte une étape (106b) pour définir une date d'échéance (C1,...,Cx) pour que la valeur courante recouvre la valeur nominale selon ladite action.

5. Procédé (100) selon l'une quelconque des revendications 3 ou 4, pour lequel l'étape (107) pour déclencher une action consiste à mettre à jour l'enregistrement dédié audit paramètre (P1,...,Px) pour y inscrire la valeur nominale (P1(t+n),...,Px(t+n)) dudit paramètre (P1,...,Px) ou la date d'échéance (C1,...,Cx) pour la valeur courante recouvre la valeur nominale selon ladite action.

6. Procédé (100) selon l'une quelconque des revendications précédentes, pour lequel :
- l'unité de traitement (UT) coopère avec un actionneur (14) pour mettre en œuvre une action déterminée ;
- l'étape (107) pour déclencher une action, consiste à élaborer une commande à destination dudit actionneur (14) pour mettre en œuvre l'action selon la distance (Δ) estimée.

7. Procédé (100) selon l'une quelconque des revendications précédentes, pour lequel :
- l'unité de traitement (UT) coopère avec une interface homme-machine de restitution (15) ;
- l'étape (107) pour déclencher une action consiste à élaborer une consigne à destination d'un utilisateur dudit système et déclencher la restitution de ladite consigne par l'interface homme-machine de restitution (15).

8. Procédé selon la revendication 2, 3 ou 4, pour lequel :
- les moyens de mémorisation (MM) comportent un deuxième enregistrement dédié à un deuxième paramètre de milieu (P2) et agencé pour mémoriser une valeur nominale (P2(t+n)) et une valeur courante (P2(t)) dudit deuxième paramètre (P2) ;
- ledit procédé (100) comporte une étape (108), postérieure à l'étape (106) pour élaborer une action, pour estimer une valeur nominale (P2(t+n)) et/ou une valeur courante (P2(t)) dudit deuxième paramètre (P2) selon l'action précédemment élaborée.

9. Procédé (100) selon l'une quelconque des revendications précédentes, comportant une étape (110) pour acquitter la date d'échéance (C1,...,Cx) d'une action, consistant à inscrire dans le champ pour mémoriser une date d'échéance (C1,...,Cx) une valeur prédéterminée caractérisant l'absence de définition de la date d'échéance, ladite étape (110) étant mise en œuvre si la valeur de la distance (Δ) entre ladite valeur nominale (P1(t+n),...,Px(t+n)) et la valeur courante (P1(t),...,Px(t)), atteste d'une distance sensiblement nulle (109-y).

10. Procédé (100) selon l'une quelconque des revendications précédentes, comportant une étape (113) de correction d'une action, antérieure à l'étape (107) pour déclencher une action, ladite étape (113) de correction étant mise en œuvre si et seulement si :
∘ la date d'échéance pour que la valeur courante recouvre la valeur nominale soit atteinte (104-n) ;
∘ la valeur de la distance (Δ) entre ladite valeur nominale (P1(t+n),...,Px(t+n)) et la valeur courante (P1(t),...,Px(t)), est non-nulle (112-n).

11. Procédé (100) selon la revendication précédente, pour lequel l'étape (113) de correction d'une action comporte une étape (113a) pour estimer une nouvelle valeur nominale (P1(t+n),...,Px(t+n)) dudit paramètre (P1,...,Px) .

12. Procédé (100) selon la revendication 10 ou 11, pour lequel l'étape (113) de correction d'une action comporte une étape (113b) pour définir la date d'échéance (C1,...,Cx) pour que la valeur courante recouvre la valeur nominale selon ladite correction d'une action.

13. Procédé (100) selon l'une quelconque des revendications précédentes, pour lequel :
- les moyens de mémorisation (MM) comportent une valeur courante d'une période précédente (P1(t-n),...,Px(t-n));
- l'étape (102) pour lire la valeur courante (P1(t),...,Px(t)) consiste en outre à lire la valeur précédente (P1(t-n),...,Px(t-n)) et calculer une nouvelle valeur courante à partir desdites valeurs lues.

14. Procédé (100) selon l'une quelconque des revendications précédentes, pour lequel :
- les moyens de mémorisation (MM) comportent une donnée d'anticipation ;
- l'étape (102) pour lire la valeur courante (P1(t),...,Px (t)) du paramètre (P1,...,Px) consiste en outre à lire la donnée d'anticipation et calculer une nouvelle valeur courante à partir de ladite valeur d'anticipation.

15. Procédé selon l'une quelconque des revendications précédentes, pour lequel :
- l'unité de traitement (UT) coopère en outre avec des moyens de capture (MC) ;
- ledit procédé (100) comporte une étape (101) préalable à l'étape (102) pour lire la valeur courante (P1(t),...,Px(t)) dudit paramètre (P1,...,Px), pour déclencher l'acquisition de ladite valeur (P1(t),...,Px(t)) par lesdits moyens de capture (MC) et inscrire ladite valeur comme valeur courante (P1(t),...,Px (t)) .

16. Produit programme d'ordinateur (P) comportant des instructions de programme exploitables par une unité de traitement (UT) d'un module électronique (ME), ladite unité de traitement (UT) coopérant avec des moyens de capture (MC) et des moyens de mémorisation, qui lorsqu'elles sont exécutées ou interprétées par ladite unité de traitement (UT), déclenchent la mise en œuvre d'un procédé maintenir un équilibre d'un paramètre physico-chimique d'un milieu selon l'une quelconque des revendications 1 à 15.

17. Module électronique (ME) comportant une unité de traitement (UT) coopérant avec des moyens de capture (MC) et des moyens de mémorisation (MM, MP), ledit module électronique (ME) étant **caractérisé en ce qu'**il comporte dans des moyens de mémorisation (MP), des instructions d'un produit programme d'ordinateur (P) selon la revendication précédente.

## Patentansprüche

1. Verfahren (100) zum Beibehalten eines Gleichgewichts eines physikalischchemischen Parameters (P1, ..., Px) eines Mediums (E), wobei das Verfahren (100) durch eine Verarbeitungseinheit (UT) eines Systems zum Regulieren des Mediums implementiert wird, wobei die Verarbeitungseinheit (UT) mit Speichermitteln (MM) zusammenwirkt, die Daten bezüglich eines Medienparameters (P1, ..., Px) abspeichern, die Speichermittel (MM) umfassend:
- einen Datensatz (RP1, ..., RPx), der dem Medienparameter (P1, ..., Px) zugeordnet ist und zum Speichern eines aktuellen Werts (P1(t),..., Px(t)) des Parameters (P1, ..., Px) und eines Nominalwerts (P1(t+n), ..., Px(t+n)) des Parameters eingerichtet ist;
das Verfahren (100) umfassend:
- einen Schritt (102) zum Lesen des aktuellen Werts (P1(t),..., Px(t)) des Parameters (P1, ..., Px);
wobei der aktuelle Wert Px(t) der Wert des Parameters Px ist, der durch Erfassungsmittel MC beziehungsweise durch die Verarbeitungseinheit UT gemessen oder berechnet wird;
- einen Schritt (103) zum Lesen des Nominalwerts (P1(t+n), ..., Px(t+n)) des Parameters (P1, ..., Px);
wobei der Nominalwert ein Ziel- oder Erwartungswert ist, der einen normalen Zustand des Mediums beschreibt;
- einen Schritt (105) zum Schätzen einer Distanz (Δ) zwischen dem Nominalwert (P1(t+n), ..., Px(t+n)) und dem aktuellen Wert (P1(t),..., Px (t));
- einen Schritt (107) zum Auslösen einer Aktion gemäß dem Wert der Distanz (Δ);
wobei das Verfahren (100) **dadurch gekennzeichnet ist, dass**:
- der Datensatz, der dem Medienparameter (P1, ..., Px) zugeordnet ist, ein Feld zum Speichern eines Verfallsdatums (C1, ..., Cx) umfasst, damit der aktuelle Wert den Nominalwert überlappt;
- wobei das Verfahren (100) umfasst:
∘ einen Schritt (104) vor dem Schritt (107) zum Auslösen einer Aktion, zum Lesen des Werts des Felds zum Speichern des Verfallsdatums (C1, ..., Cx);
∘ den Schritt (107) zum Auslösen einer Aktion gemäß der Distanz (Δ), die genau dann implementiert wird, wenn:
▪ der Wert des Feldes zum Speichern des Verfallsdatums (C1, ..., Cx) aus einem zuvor bestimmten Wert besteht, der die fehlende Definition des Verfallsdatums (104-0) kennzeichnet, damit der aktuelle Wert den Nominalwert überlappt.

2. Verfahren (100) nach dem vorstehenden Anspruch, umfassend einen Schritt (106) vor dem Schritt (107) zum Auslösen einer Aktion zum Ausarbeiten der Aktion gemäß dem Wert der Distanz (Δ).

3. Verfahren (100) nach Anspruch 2, wobei der Schritt (106) zum Ausarbeiten einer Aktion einen Schritt (106a) zum Schätzen eines Nominalwerts (P1(t+n), ..., Px(t+n)) des Parameters (P1, ..., Px) umfasst.

4. Verfahren (100) nach Anspruch 2 oder 3, wobei der Schritt (106) zum Ausarbeiten einer Aktion einen Schritt (106b) zum Definieren eines Verfallsdatums (C1, ..., Cx) umfasst, damit der aktuelle Wert den Nominalwert gemäß der Aktion überlappt.

5. Verfahren (100) nach einem der Ansprüche 3 oder 4, wobei der Schritt (107) zum Auslösen einer Aktion darin besteht, den Datensatz zu aktualisieren, der dem Parameter (P1, ..., Px) zugeordnet ist, zum Aufzeichnen darin des Nennwerts (P1(t+n), ..., Px(t+n)) des Parameters (P1, ..., Px) oder des Verfallsdatums (C1, ..., Cx), damit der aktuelle Wert den Nominalwert gemäß der Aktion überlappt.

6. Verfahren (100) nach einem der vorstehenden Ansprüche, wobei:
- die Verarbeitungseinheit (UT) mit einem Aktuator (14) zum Implementieren einer bestimmten Aktion zusammenwirkt;
- der Schritt (107) zum Auslösen einer Aktion darin besteht, einen Befehl für den Aktuator (14) zum Implementieren der Aktion gemäß der geschätzten Distanz (Δ) auszuarbeiten.

7. Verfahren (100) nach einem der vorstehenden Ansprüche, wobei:
- die Verarbeitungseinheit (UT) mit einer Mensch-Maschine-Ausgabeschnittstelle (15) zusammenwirkt;
- der Schritt (107) zum Auslösen einer Aktion darin besteht, eine Anweisung für einen Benutzer des Systems auszuarbeiten und die Ausgabe der Anweisung durch die Mensch-Maschine-Ausgabeschnittstelle (15) auszulösen.

8. Verfahren nach Anspruch 2, 3 oder 4, wobei:
- die Speichermittel (MM) einen zweiten Datensatz umfassen, der einem zweiten Medienparameter (P2) zugeordnet ist und zum Speichern eines Nominalwerts (P2(t+n)) und eines aktuellen Werts (P2(t)) des zweiten Parameters (P2) eingerichtet ist;
- das Verfahren (100) einen Schritt (108), nach dem Schritt (106) zum Ausarbeiten einer Aktion, zum Schätzen eines Nominalwerts (P2(t+n)) und/oder eines aktuellen Werts (P2(t)) des zweiten Parameters (P2) gemäß der zuvor ausgearbeiteten Aktion umfasst.

9. Verfahren (100) nach einem der vorstehenden Ansprüche, umfassend einen Schritt (110) zum Bestätigen des Verfallsdatums (C1, ..., Cx) einer Aktion, der darin besteht, in dem Feld zum Speichern eines Verfallsdatums (C1, ..., Cx) einen zuvor bestimmten Wert aufzuzeichnen, der eine fehlende Definition des Verfallsdatums kennzeichnet, wobei der Schritt (110) implementiert wird, falls der Wert der Distanz (Δ) zwischen dem Nominalwert (P1 (t+n), ..., Px(t+n)) und dem aktuellen Wert (P1(t), ..., Px(t)) eine Distanz von im Wesentlichen null nachweist (109-y).

10. Verfahren (100) nach einem der vorstehenden Ansprüche, umfassend einen Schritt (113) zum Korrigieren einer Aktion vor dem Schritt (107) zum Auslösen einer Aktion, wobei der Korrekturschritt (113) genau dann implementiert wird, wenn:
∘ das Verfallsdatum, damit der aktuelle Wert den Nominalwert überlappt, erreicht ist (104-n);
∘ der Wert der Distanz (Δ) zwischen dem Nominalwert (P1(t+n), ..., Px(t+n)) und dem aktuellen Wert (P1(t), ..., Px(t)) ungleich null ist (112-n).

11. Verfahren (100) nach dem vorstehenden Anspruch, wobei der Schritt (113) zum Korrigieren einer Aktion einen Schritt (113a) zum Schätzen eines neuen Nominalwerts (P1(t+n), ..., Px(t+n)) des Parameters (P1, ..., Px) umfasst.

12. Verfahren (100) nach Anspruch 10 oder 11, wobei der Schritt (113) zum Korrigieren einer Aktion einen Schritt (113b) zum Definieren des Verfallsdatums (C1, ..., Cx) umfasst, damit der aktuelle Wert den Nominalwert gemäß der Korrektur einer Aktion überlappt.

13. Verfahren (100) nach einem der vorstehenden Ansprüche, wobei:
- die Speichermittel (MM) einen aktuellen Wert aus einer vorherigen Periode (P1(tn), ..., Px(tn)) umfassen;
- der Schritt (102) zum Lesen des aktuellen Werts (P1(t), ..., Px(t)) ferner aus dem Lesen des vorherigen Werts (P1(tn), ..., Px(tn)) und dem Berechnen eines neuen aktuellen Werts aus den gelesenen Werten umfasst.

14. Verfahren (100) nach einem der vorstehenden Ansprüche, wobei:
- die Speichermittel (MM) ein Vorgriffsdatenelement umfassen;
- der Schritt (102) zum Lesen des aktuellen Werts (P1(t), ..., Px(t)) des Parameters (P1, ..., Px) ferner aus dem Lesen des Vorgriffsdatenelements und dem Berechnen eines neuen aktuellen Werts aus dem Vorgriffswert besteht.

15. Verfahren nach einem der vorstehenden Ansprüche, wobei:
- die Verarbeitungseinheit (UT) ferner mit Erfassungsmitteln (MC) zusammenwirkt;
- das Verfahren (100) einen Schritt (101) vor Schritt (102) zum Lesen des aktuellen Werts (P1(t), ..., Px(t)) des Parameters (P1, ..., Px), zum Auslösen der Beschaffung des Werts (P1(t), ..., Px(t)) durch die Erfassungsmittel (MC) und zum Schreiben des Werts als aktuellen Wert (P1(t), ..., Px(t)) umfasst.

16. Computerprogrammprodukt (P), umfassend Programminstruktionen, die durch eine Verarbeitungseinheit (UT) eines elektronischen Moduls (ME) verwendbar sind,
wobei die Verarbeitungseinheit (UT) mit Erfassungsmitteln (MC) und Speichermitteln zusammenwirkt, die, wenn sie durch die Verarbeitungseinheit (UT) ausgeführt oder interpretiert werden, die Implementierung eines Verfahrens zum Beibehalten eines Gleichgewichts eines physikalisch-chemischen Parameters eines Mediums gemäß einem der Ansprüche 1 bis 15 auslösen.

17. Elektronisches Modul (ME), umfassend eine Verarbeitungseinheit (UT), die mit Erfassungsmitteln (MC) und Speichermitteln (MM, MP) zusammenwirkt, wobei das elektronische Modul (ME) **dadurch gekennzeichnet ist, dass** es in Speichermitteln (MP) Instruktionen eines Computerprogrammprodukts (P) nach dem vorstehenden Anspruch umfasst.

## Claims

1. Method (100) for maintaining an equilibrium of a physico-chemical parameter (P1,...,Px) of a medium (E), said method (100) being implemented by a processing unit (UT) of a system for regulating said medium, said processing unit (UT) cooperating with storage means (MM) recording data related to a medium parameter (P1,...,Px), said storage means (MM) including:
- a record (RP1,...,RPx) dedicated to said medium parameter (P1,...,Px) and arranged to store a current value (P1(t),...,Px(t)) of said parameter (P1,...,Px) and a nominal value (P1(t+n),...,Px(t+n)) of said parameter;
said method (100) including:
- a step (102) for reading the current value (P1(t),...,Px(t)) of said parameter (P1,...,Px);
the current value Px(t) being the value of said parameter Px measured or calculated respectively by sensing means MC or by the processing unit UT;
- a step (103) for reading the nominal value (P1(t+n),...,Px(t+n)) of said parameter (P1,...,Px);
the nominal value being a target or expected value describing a normal state of the medium;
- a step (105) for estimating a distance (Δ) between said nominal value (P1(t+n),...,Px(t+n)) and the current value (P1(t),...,Px(t));
- a step (107) for triggering an action according to the value of said distance (Δ);
said method (100) being **characterized in that:**
- the record dedicated to said medium parameter (P1,...,Px) includes a field for storing a due date (C1,...,Cx) for the current value to recover the nominal value;
- said method (100) includes:
∘ a step (104), prior to the step (107) for triggering an action, for reading the value of the field for storing the due date (C1,...,Cx);
∘ the step (107) for triggering an action according to said distance (Δ) being implemented if and only if:
▪ the value of the field for storing the due date (C1,...,Cx) consists of a predetermined value characterizing the absence of definition of the due date (104-0) for the current value to recover the nominal value.

2. Method (100) according to the preceding claim, including a step (106), prior to the step (107) for triggering an action, for elaborating said action according to the value of said distance (Δ).

3. Method (100) according to claim 2, wherein the step (106) for elaborating an action includes a step (106a) for estimating a nominal value (P1(t+n),...,Px(t+n)) of said parameter (P1,...,Px).

4. Method (100) according to claim 2 or 3, wherein the step (106) for elaborating an action includes a step (106b) for defining a due date (C1,...,Cx) for the current value to recover the nominal value according to said action.

5. Method (100) according to claim 3 or claim 4, wherein the step (107) for triggering an action consists in updating the record dedicated to said parameter (P1,...,Px) to register therein the nominal value (P1(t+n),...,Px(t+n)) of said parameter (P1,...,Px) or the due date (C1,...,Cx) for the current value to recover the nominal value according to said action.

6. Method (100) according to any of the preceding claims, wherein:
- the processing unit (UT) cooperates with an actuator (14) to implement a specific action;
- the step (107) for triggering an action consists in elaborating a command for said actuator (14) to implement the action according to the estimated distance (Δ).

7. Method (100) according to any of the preceding claims, wherein:
- the processing unit (UT) cooperates with a human-machine rendering interface (15);
- the step (107) for triggering an action consists in elaborating an instruction for a user of said system and triggering the rendering of said instruction by the human-machine rendering interface (15).

8. Method according to claim 2, 3 or 4, wherein:
- the storage means (MM) include a second record dedicated to a second medium parameter (P2) and arranged to store a nominal value (P2(t+n)) and a current value (P2(t)) of said second parameter (P2);
- said method (100) includes a step (108), subsequent to the step (106) for elaborating an action, for estimating a nominal value (P2(t+n)) and/or a current value (P2(t)) of said second parameter (P2) according to the previously elaborated action.

9. Method (100) according to any of the preceding claims, including a step (110) for acknowledging the due date (C1,...,Cx) of an action, consisting in registering in the field for storing a due date (C1,...,Cx) a predetermined value characterizing the absence of definition of the due date, said step (110) being implemented if the value of the distance (Δ) between said nominal value (P1(t+n),...,Px(t+n)) and the current value (P1(t),...,Px(t)) attests to a substantially zero distance (109-y).

10. Method (100) according to any of the preceding claims, including a step (113) for correcting an action, prior to the step (107) for triggering an action, said correction step (113) being implemented if and only if:
∘ the due date for the current value to recover the nominal value is reached (104-n);
∘ the value of the distance (Δ) between said nominal value (P1(t+n),...,Px(t+n)) and the current value (P1(t),...,Px(t)) is nonzero (112-n).

11. Method (100) according to the preceding claim, wherein the step (113) for correcting an action includes a step (113a) for estimating a new nominal value (P1(t+n),...,Px(t+n)) of said parameter (P1,...,Px).

12. Method (100) according to claim 10 or 11, wherein the step (113) for correcting an action includes a step (113b) for defining the due date (C1,...,Cx) for the current value to recover the nominal value according to said correction of an action.

13. Method (100) according to any of the preceding claims, wherein:
- the storage means (MM) include a current value of a previous period (P1(t-n),...,Px(t-n));
- the step (102) for reading the current value (P1(t),...,Px(t)) further consists in reading the previous value (P1(t-n),...,Px(t-n)) and calculating a new current value from said read values.

14. Method (100) according to any of the preceding claims, wherein:
- the storage means (MM) include anticipation data;
- the step (102) for reading the current value (P1(t),...,Px(t)) of the parameter (P1,...,Px) further consists in reading the anticipation data and calculating a new current value from said anticipation value.

15. Method according to any of the preceding claims, wherein:
- the processing unit (UT) further cooperates with sensing means (MC);
- said method (100) includes a step (101), prior to the step (102) for reading the current value (P1(t),...,Px(t)) of said parameter (P1,...,Px), for triggering the acquisition of said value (P1(t),...,Px(t)) by said sensing means (MC) and registering said value as the current value (P1(t),...,Px(t)).

16. Computer program product (P) including program instructions that can be used by a processing unit (UT) of an electronic module (ME),
said processing unit (UT) cooperating with sensing means (MC) and storage means, which, when executed or interpreted by said processing unit (UT), trigger the implementation of a method for maintaining an equilibrium of a physico-chemical parameter of a medium according to any of claims 1 to 15.

17. Electronic module (ME) including a processing unit (UT) cooperating with sensing means (MC) and storage means (MM, MP), said electronic module (ME) being **characterized in that** it includes, in storage means (MP), instructions of a computer program product (P) according to the preceding claim.
